(19) Europäisches Patentamt
European Patent Office
Office européen des brevets

(11) Veröffentlichungsnummer: **0 115 823**
**B1**

(12) # EUROPÄISCHE PATENTSCHRIFT

(45) Veröffentlichungstag der Patentschrift: **20.05.87**

(21) Anmeldenummer: **84100796.6**

(22) Anmeldetag: **26.01.84**

(51) Int. Cl.[4]: **C 07 C 153/017**, C 07 C 153/09, C 07 D 213/64, A 01 N 39/02, A 01 N 39/04, A 01 N 43/40

(54) **Substituierte Alkanthiocarbonsäure-Derivate.**

(30) Priorität: **08.02.83 DE 3304204**

(43) Veröffentlichungstag der Anmeldung: **15.08.84 Patentblatt 84/33**

(45) Bekanntmachung des Hinweises auf die Patenterteilung: **20.05.87 Patentblatt 87/21**

(84) Benannte Vertragsstaaten: **AT BE CH DE FR GB IT LI NL SE**

(56) Entgegenhaltungen:
**EP-A-0 002 204**
**DE-A-1 939 010**
**DE-A-2 758 002**

(73) Patentinhaber: **BAYER AG, Konzernverwaltung RP Patentabteilung, D-5090 Leverkusen 1 Bayerwerk (DE)**

(72) Erfinder: **Förster, Heinz, Dr., Am Eckbusch 47, D-5600 Wuppertal 1 (DE)**
Erfinder: **Eue, Ludwig, Dr., Paul- Klee- Strasse 36, D-5090 Leverkusen (DE)**
Erfinder: **Schmidt, Robert R., Dr., Im Waldwinkel 110, D-5060 Bergisch- Gladbach 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



LIBER, STOCKHOLM 1987

**Beschreibung**

Die Erfindung betrifft neue, substituierte Alkanthiocarbonsäure-Derivate, ein Verfahren zu deren Herstellung sowie ihre Verwendung als Herbizide.

Es ist bereits bekannt geworden, daß zahlreiche Phenoxypropionsäure-Derivate herbizide Eigenschaften besitzen (vgl. DE-OS 2 223 894). So kann z.B. der 2-[4-(2,4-Di-chlor-phenoxy)-phenoxy]-propionsäure-methylester zur Unkrautbekämpfung eingesetzt werden. Die Wirkung dieses Stoffes ist jedoch insbesondere bei einigen Gräsern und beim Einsatz niedriger Aufwandmengen nicht immer ausreichend.

Es wurden nun neue substituierte Alkanthiocarbonsäure-Derivate der Formel

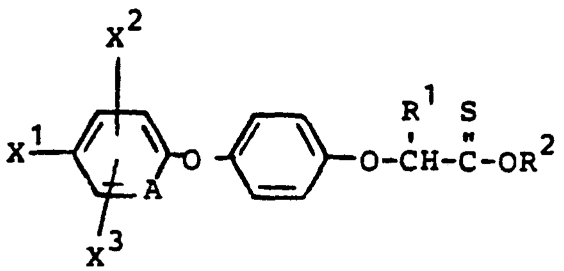
(I)

in welcher

A für Stickstoff oder eine CH-Gruppierung steht,

$X^1$ für Wasserstoff, Trifluormethyl oder Chlor,

$X^2$ für Wasserstoff, Triflourmethyl oder Chlor,

$X^3$ für Wasserstoff, Trifluormethyl oder Chlor,

$R^1$ für Wasserstoff oder Methyl und

$R^2$ für Wasserstoff, gegebenenfalls durch Hydroxy, Halogen, wie Fluor, Chlor oder-Brom, $C_1$-$C_4$-Alkoxy, $C_1$-$C_4$-Alkylthio, $C_3$-$C_6$-Cycloalkyl, Amino, Alkylamino mit 1 bis 1 Kohlenstoffatomen, Dialkylamino mit 1 bis 4 Kohlenstoffatomen pro Alkylrest und/oder über Stickstoff gebundene, gesättigte, 5- oder 6-glied-rige Heterocyclen, die bis zu 3 Stickstoff- und/oder Sauerstoffatoms enthalten, substituiertes Alkyl mit 1 bis 6 Kohlenstoffatomen,

für gegebehenfalls durch Fluor, Chlor, Brom und/oder lod substituiertes Alkenyl,

für gegebenenfalls durch Fluor, Chlor, Brom und/oder lod substituiertas Alkinyl,

für gegebenenfalls durch Halogen, $C_1$-$C_4$-Alkyl, $C_1$-$C_4$-Alkylthio, $C_1$-$C_4$-Alkoxy, Cyano und/oder Nitro substituiertes phenyl,

für gegebenenfalls durch Halogen, $C_1$-$C_4$-Alkyl, $C_1$-$C_4$-Alkylthio, $C_1$-$C_4$-Alkoxy, Cyano und/oder Nitro substituiertes Benzyl,

für gegebenenfalls im Phenylteil durch Halogen $C_1$-$C_4$-Alkyl, $C_1$-$C_4$-Alkylthio, $C_1$-$C_4$-Alkoxy, Cyano und/oder Nitro substituiertes Phenylethyl,

für gegebenenfalls im phenylteil durch Halogen, $C_1$-$C_4$-Alkyl, $C_1$-$C_4$-Alkylthio, $C_1$-$C_4$-Alkoxy, Cyano und/oder Nitro substituiertes Phenyl-($C_1$-$C_4$)-alkoxy-($C_1$-$C_4$)-alkyl oder

für gegebenenfalls durch Alkyl mit 1 bis 4 Kohlenstoffatomen und/oder Halogen substituiertes Cycloalkyl mit 3 bis 6 Kohlenstoffatomen,

gefunden.

Diejenigen substituierten Alkanthiocarbonsäure-Derivate der Formel (I), in denen $R^1$ für Methyl steht, enthalten ein asymmetrisches Kohlenstoffatom in der Seitenkette und können deshaib in zwei enantiomeren Formen vorliegen. Die Erfindung betrifft sowohl die jeweiligen Racemate als auch die R- und S-Enantiomeren.

Weiterhin wurde gefunden, daß man die substituierten Alkanthiocarbonsäure-Derivate der Formel (I) erhält, wenn man phenoxyalkancarbonsäure-Derivate der Formel

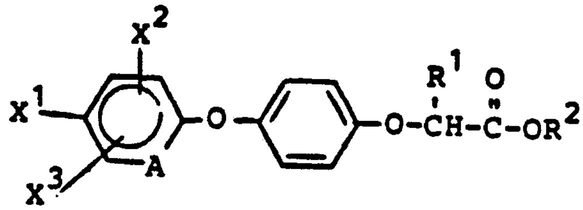
(II)

in welcher

A, $X^1$, $X^2$, $X^3$, $R^1$ und $R^2$ die oben angegebene Bedeutung haben,

mit Schwefelungsmitteln gegebenenfalls in Gegenwart eines Verdünnungsmittels umsetzt.

Schließlich wurde gefunden, daß sich die neuen substituierten Alkanthiocarbonsäure-Derivate der Formel (I) durch eine hervorragende herbizide Wirksamkeit auszeichnen.

Überraschenderweise besitzen die erfindungsgemäßen substituierten Alkanthiocarbonsäure-Derivate der Formel (I) bessere herbizide Eigenschaften als der aus dem Stand der Technik bekannte 4-[4-(2,4-Dichlorphenoxy)-phenoxy]propionsäuremethylester, welcher ein hoch wirksamer und konstitutionell ähnlicher Wirkstoff gleicher Wirkungsart ist. Vor allem lassen sich mit Hilfe der erfindungsgemäßen Wirkstoffe der Formel (I) einige Ungräser, die insbesondere in Reis vorkommen und von dem 4-[4-(2,4-Dichlor-phenoxy)-phenoxy]-propionsäuremethylester nicht erfaßt werden, wirksam bekämpfen.

Die erfindungsgemäßen substituierten Alkanthiocarbonsäure-Derivate sind durch die Formel (I) eindeutig definiert.

Eine bevorzugte Gruppe von erfindungsgemäßen Verbindungen sind diejenigen Stoffe der Formel-(I), in denen A für eine CH-Gruppe steht, $X^1$ für Trifluormethyl steht, $X^2$ und $X^3$ für Wasserstoff oder Chlor stehen, $R^1$ für Methyl steht und $R^2$ für Methyl oder Ethyl steht.

Eine weitere Gruppe von bevorzugten erfindungsgemäßen Verbindungen sind diejenigen Stoffe der Formel (I), in denen-A für Stickstoff steht, $X^1$ für Chlor oder Trifluormethyl steht, $X^2$ für Wasserstoff oder Chlor steht, $X^3$ für Wasserstoff steht, $R^1$ für Methyl steht und $R^2$ für Methyl oder Ethyl steht.

Als weitere bevorzugte Gruppen von erfindungsgemäßen Verbindungen der Formel (I) seien die in den folgenden Tabellen formelmäßig aufgeführten Verbindungen genannt:

**Tabelle 1**

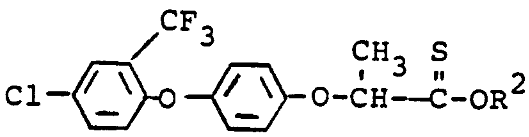

(Ia)

| $R^2$ | $R^2$ |
|---|---|
| $n-C_3H_7$ | $-CH_2-CH(OC_2H_5)_2$ |
| $i-C_3H_7$ | $-CH_2-CCl_3$ |
| $n-C_4H_9$ | $-CH_2-CH_2-$ 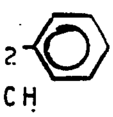 |
| tert.$-C_4H_9$ | $-CH(CH_3)-C\equiv CH$ |
| $-CH_2-CH=CH_2$ | $-CH(CH_2Cl)_2$ |
| $-CH_2-C\equiv CH$ | $-CH(CH_3)-$ 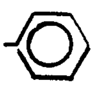 |
| $-CH_2-CH_2-OCH_3$ | $-CH_2-CF_3$ |
| $-CH_2-CH_2-SCH_3$ | $-CH_3$ |
| $-CH_2-CH_2-O-CH_2-$ 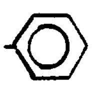 | $-C_2H_5$ |
| $-CH_2-CH_2-Cl$ | $-CH_2-CH_2-S-C_2H_5$ |
| $-CH_2-CH_2-N(CH_3)_2$ | |
| $-CH_2-$ 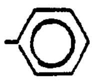 | |

**Tabelle 2**

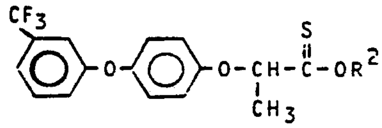

(I b)

| $R^2$ | $R^2$ |
|---|---|
| $n-C_3H_7$ | $-CH_2-CH(OC_2H_5)_2$ |
| $i-C_3H_7$ | $-CH_2-CCl_3$ |
| $n-C_4H_9$ | 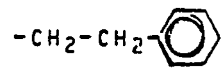 |
| $tert.-C_4H_9$ | $-CH_2-CH_2-S-C_2H_5$ |
| $-CH_2-CH=CH_2$ | $-CH(CH_3)-C\equiv CH$ |
| $-CH_2-C\equiv CH$ | $-CH(CH_2Cl)_2$ |
| $-CH_2-CH_2-OCH_3$ | 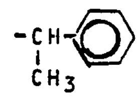 |
| $-CH_2-CH_2-SCH_3$ | $-CH_2-CF_3$ |
| 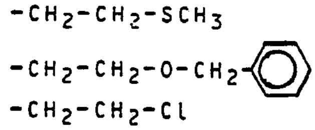 | $-CH_3$ |
| $-CH_2-CH_2-Cl$ | $-C_2H_5$ |
| $-CH_2-CH_2-N(CH_3)_2$ | |
| 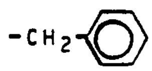 | |

**Tabelle 3**

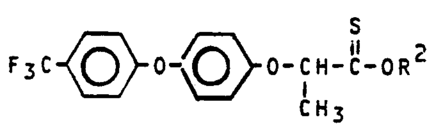

(I c)

| $R^2$ | $R^2$ |
|---|---|
| $n-C_3H_7$ | $-CH_2-CH(OC_2H_5)_2$ |
| $i-C_3H_7$ | $-CH_2-CCl_3$ |
| $n-C_4H_9$ | 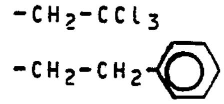 |
| $tert.-C_4H_9$ | $-CH_2-CH_2-S-C_2H_5$ |
| $-CH_2-CH=CH_2$ | $-CH(CH_3)-C\equiv CH$ |
| $-CH_2-C\equiv CH$ | $-CH(CH_2Cl)_2$ |
| $-CH_2-CH_2-OCH_3$ | 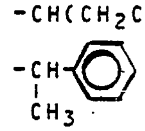 |
| $-CH_2-CH_2-SCH_3$ | $-CH_2-CF_3$ |
| 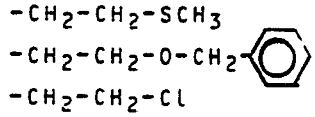 | $-CH_3$ |
| $-CH_2-CH_2-Cl$ | $-C_2H_5$ |
| $-CH_2-CH_2-N(CH_3)_2$ | |
| 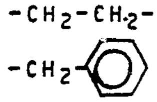 | |

**Tabelle 4**

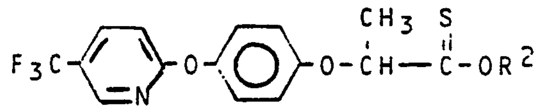 (I d)

| $R^2$ | $R^2$ |
|---|---|
| $n-C_3H_7$ | $-CH_2-CH(OC_2H_5)_2$ |
| $i-C_3H_7$ | $-CH_2-CCl_3$ |
| $n-C_4H_9$ | $-CH_2-CH_2-$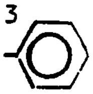 |
| $tert.-C_4H_9$ | $-CH_2-CH_2-S-C_2H_5$ |
| $-CH_2-CH=CH_2$ | $-\underset{CH_3}{CH}-C\equiv CH$ |
| $-CH_2-C\equiv CH$ | $-CH(CH_2Cl)_2$ |
| $-CH_2-CH_2-OCH_3$ | $-\underset{CH_3}{CH}-$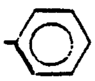 |
| $-CH_2-CH_2-SCH_3$ | $-CH_2-CF_3$ |
| $-CH_2-CH_2-O-CH_2-$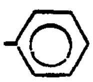 | $-CH_3$ |
| $-CH_2-CH_2-Cl$ | $-C_2H_5$ |
| $-CH_2-CH_2-N(CH_3)_2$ | |
| $-CH_2-$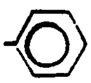 | |

**Tabelle 5**

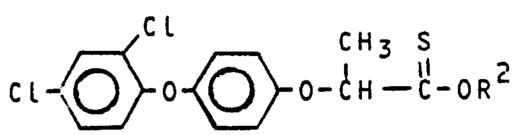 (I e)

| $R^2$ | $R^2$ |
|---|---|
| $n-C_3H_7$ | $-CH_2-CH(OC_2H_5)_2$ |
| $i-C_3H_7$ | $-CH_2-CCl_3$ |
| $n-C_4H_9$ | $-CH_2-CH_2-$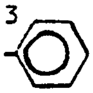 |
| $tert.-C_4H_9$ | $-CH_2-CH_2-S-C_2H_5$ |
| $-CH_2-CH=CH_2$ | $-\underset{CH_3}{CH}-C\equiv CH$ |
| $-CH_2-C\equiv CH$ | $-CH(CH_2Cl)_2$ |
| $-CH_2-CH_2-OCH_3$ | $-\underset{CH_3}{CH}-$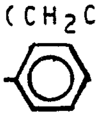 |
| $-CH_2-CH_2-SCH_3$ | $-CH_2-CF_3$ |
| $-CH_2-CH_2-O-CH_2-$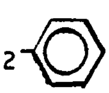 | $-CH_3$ |
| $-CH_2-CH_2-Cl$ | $-C_2H_5$ |
| $-CH_2-CH_2-N(CH_3)_2$ | |
| $-CH_2-$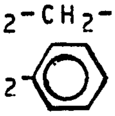 | |

**Tabelle 6**

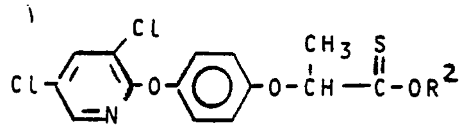

(I f)

| $R^2$ | $R^2$ |
|---|---|
| $n-C_3H_7$ | $-CH_2-CH(OC_2H_5)_2$ |
| $i-C_3H_7$ | $-CH_2-CCl_3$ |
| $n-C_4H_9$ | $-CH_2-CH_2-$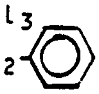 |
| $tert.-C_4H_9$ | $-CH_2-CH_2-S-C_2H_5$ |
| $-CH_2-CH=CH_2$ | $-CH-C\equiv CH$<br>$\vert$<br>$CH_3$ |
| $-CH_2-C\equiv CH$ | $-CH(CH_2Cl)_2$ |
| $-CH_2-CH_2-OCH_3$ | $-CH$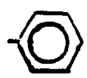<br>$\vert$<br>$CH_3$ |
| $-CH_2-CH_2-SCH_3$ | $-CH_2-CF_3$ |
| $-CH_2-CH_2-O-CH_2-$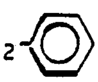 | $-CH_3$ |
| $-CH_2-CH_2-Cl$ | $-C_2H_5$ |
| $-CH_2-CH_2-N(CH_3)_2$ | |
| $-CH_2-$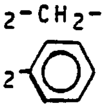 | |

**Tabelle 7**

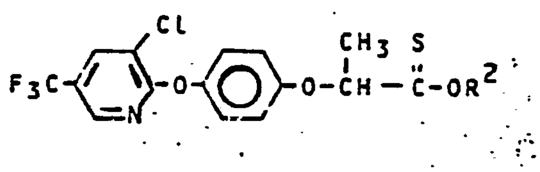

(I g)

| $R^2$ | $R^2$ |
|---|---|
| $n-C_3H_7$ | $-CH_2-CH(OC_2H_5)_2$ |
| $i-C_3H_7$ | $-CH_2-CCl_3$ |
| $n-C_4H_9$ | $-CH_2-CH_2-$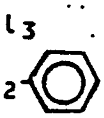 |
| $tert.-C_4H_9$ | $-CH_2-CH_2-S-C_2H_5$ |
| $-CH_2-CH=CH_2$ | $-CH-C\equiv CH$<br>$\vert$<br>$CH_3$ |
| $-CH_2-C\equiv CH$ | $-CH(CH_2Cl)_2$ |
| $-CH_2-CH_2-OCH_3$ | $-CH-$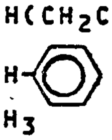<br>$\vert$<br>$CH_3$ |
| $-CH_2-CH_2-SCH_3$ | $-CH_2-CF_3$ |
| $-CH_2-CH_2-O-CH_2-$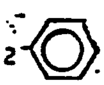 | $-CH_3$ |
| $-CH_2-CH_2-Cl$ | $-C_2H_5$ |
| $-CH_2-CH_2-N(CH_3)_2$ | |
| $-CH_2-$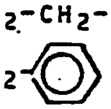 | |

Bevorzugt sind auch die R-Enantiomeren der zuvor genannten Verbindungen.

Verwendet man 2-[4-(4-Chlor-2-trifluormethyl-phenoxy)-phenoxy]-propionsäure-ethylester als Ausgangsstoff und 2,4-Bis(4-Methoxy-phenyl)-1,3,2,4-dicyclothiaphosphan-2,4-disulfid als Schwefelungsmittel, so kann der Verlauf des erfindungsgemäßen Verfahrens durch das folgende Formelschema wiedergegeben werden:

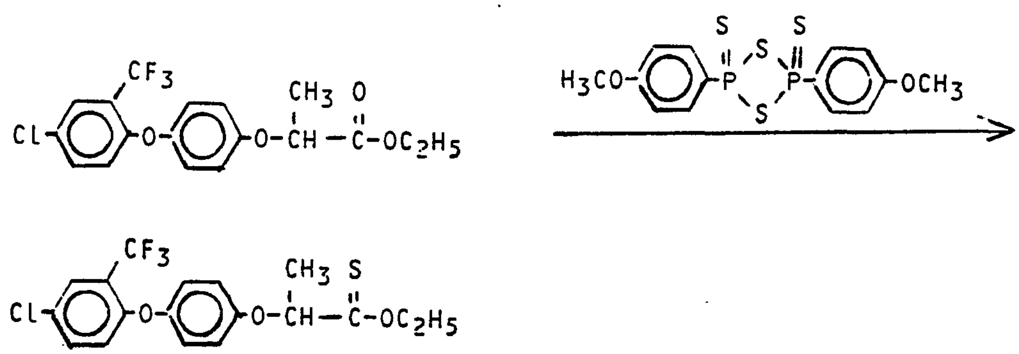

Die bei der Durchführung des erfindungsgemäßen Verfahrens als Ausgangsstoffe benötigten Phenoxyalkancarbonsäure-Derivate sind durch die Formel (II) eindeutig definiert. In dieser Formel haben A, $X^1$, $X^2$, $X^3$, $R^1$ und $R^2$ vorzugsweise diejenigen Bedeutungen, die bereits im Zusammenhang mit der Beschreibung der erfindungsgemäßen Stoffe der Formel (I) vorzugsweise für diese Reste genannt wurden.

Als Beispiele für Phenoxyalkancarbonsäure-Derivate der Formel (II) seien diejenigen Stoffe genannt, die den in den Tabellen 1 bis 7 aufgeführten Alkanthiocarbonsäure-Derivaten der Formel (I) entsprechen.

Die Phenoxyalkancarbonsäure-Derivate der Formel (II) sind bekannt oder lassen sich nach bekannten Methoden in einfacher Weise herstellen (vgl. DE-OS 32 19 789, DE-OS 32 21 214, DE-OS 32 19 821, BE-PS 862 325, BE-PS 868 875, EP-OS 483, EP-OS 17 767, EP-OS 1 473, US-PS 4 301 295 und DE-OS 29 46 652).

Als Schwefelungsmittel kommen bei der Durchführung des erfindungsgemäßen Verfahrens alle diejenigen Reagenzien in Betracht, die geeignet sind, um C=O-Gruppen in C=S-Gruppen zu überführen. Bevorzugt verwendbar ist Phosphorpentasulfid, gegebenenfalls auf Trägerstoffen wie zum Beispiel Magnesiumoxid. Weiterhin bevorzugt verwendbar ist 2,4-Bis(4-methoxyphenyl)-1,3,2,4-dicyclothiaphosphan-2,4-disulfid der Formel

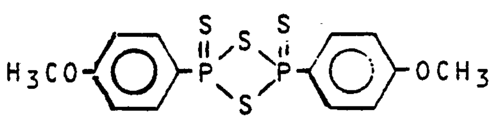

(III).

Diese Schwefelungsmittel sind bekannt oder lassen sich nach bekannten Verfahren in einfacher Weise herstellen (vgl. DE-OS 25 11 230, Bull. Soc. Chim. Belg. 1978, 87(3), 229-238 und Chem. Abstr. 89, 108 050 t).

Zur Herstellung der R- und S-Enantiomeren derjenigen substituierten Alkanthiocarbonsäure-Derivate der Formel (I), in denen $R^1$ für Methyl steht, werden die entsprechenden optisch aktiven Phenoxyalkancarbonsäure-Derivate der Formel

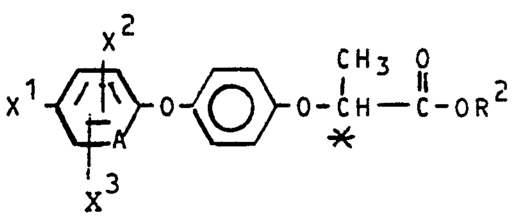

(IIa)

in welcher

A, $X^1$, $X^2$, $X^3$ und $R^2$ die oben angegebene Bedeutung haben, mit Schwefelungsmitteln nach dem erfindungsgemäßen Verfahren umgesetzt.

Die optisch aktiven Phenoxyalkancarbonsäure-Derivate der Formel (IIa) sind ebenfalls bekannt oder lassen sich nach bekannten Methoden herstellen (vgl. oben angegebene Literatur). In der Formel (IIa) ist das asymmetrisch substituierte Kohlenstoffatom durch ein (*) gekennzeichnet.

Das erfindungsgemäße Verfahren zur Herstellung der neuen Stoffe der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Chlorbenzol und o-Dichlorbenzol, Ether, wie Dibutylether, Glycoldimethylether und Diglycoldimethylether, Tetrahydrofuran und Dioxan.

Die Reaktionstemperaturen können bei dem erfindungsgemäßen Verfahren innerhalb eines größeren Bereiches variiert werden. Im allgemeinen arbeitet man jeweils bei Temperaturen zwischen 20°C und 190°C, vorzugsweise zwischen 110° und 160°C.

Das erfindungsgemäße Verfahren wird im allgemeinen unter Normaldruck durchgeführt. Es ist jedoch auch möglich, unter erhöhtem oder vermindertem Druck zu arbeiten.

Bei der Durchführung des erfindungsgemäßen Verfahrens werden die Ausgangsstoffe im allgemeinen in etwa äquimolaren Mengen eingesetzt. Es ist jedoch auch möglich, eine der beiden Komponenten in einem größeren Überschuß einzusetzen. Die Reaktion wird im allgemeinen in einem geeigneten Verdünnungsmittel durchgeführt, und das Reaktionsgemisch wird mehrere Stunden bei der erforderlichen Temperatur gerührt. Die Aufarbeitung erfolgt bei diesem Verfahren nach üblichen Methoden.

Bei der Herstellung von optischen Isomeren der substituierten Alkanthiocarbonsäure-Derivate der Formel (I) geht man zweckmäßigerweise so vor, daß man zunächst die jeweils zugrunde liegenden Phenoxyalkancarbonsäure-Derivate der Formel (IIa) nach der in der DE-OS 27 58 002 beschriebenen Methode herstellt und diese Stoffe der Formel (IIa) dann nach dem erfindungsgemäßen Verfahren in die entsprechenden Thioverbindungen überführt.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z. B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat.
Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.
Die Formulierungen enthalten im allgemeinen zwischen 0,1 und 95 Gewichtsprozent Wirkstoff, vorzugsweise zwischen 0,5 und 90 %.
Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.
Für die Mischungen kommen bekannte Herbizide wie z.B. 1-Amino-6-ethylthio-3-(2,2-dimethylpropyl)-1,3,5-tria-zin-2,4(1H,3H)-dion oder N-(2-Benzthiazolyl)-N,N'-di-methyl-harnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-di-methylethyl)-3-methylthio-1,2,4-triazin-5(4H)-on zur Unkrautbekämpfung in Sojabohnen, in Frage. Einige Mischungen zeigen überraschenderweise auch synergistische Wirkung.
Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.
Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.
Die erfindungsgemäßen Wirkstoffe können sowohl vor als auch nach dem Auflaufen der Pflanzen appliziert werden.
Sie können auch vor der Saat in den Boden eingearbeitet werden.
Die angewandte Wirkstoffmenge kann in einem größeren Bereich schwanken. Sie hängt im wesentlichen von der Art des gewänschten Effektes ab. Im allgemeinen die Aufwandmengen zwischen 0,001 und 10 kg Wirkstoff pro Hektar Bodenfläche, vorzugsweise zwischen 0,01 und 5 kg pro ha.
Die Herstellung und die Verwendung der erfindungsgemäßen Wirkstoffe geht aus den nachfolgenden Beispielen hervor.

**Herstellungsbeispiele**:

**Beispiel 1**

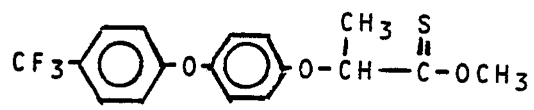

In 100 ml Xylol wurden 17 g (0,05 Mol) 2-[4-(4-Trifluor-methyl-phenoxy)-phenoxy]-propionsäuremethylester und 23,4 g (0,06 Mol) 2,4-Bis(4-methoxyphenyl)-1,3,2,4-dicyclo-thiaphosphan-2,4-disulfid 28 Stunden unter Rückfluß erhitzt. Anschließend wurde das Lösungsmittel im Wasserstrahlvakuum entfernt, der Rückstand in heißem Ligroin gelöst und mit Aktivkohle verrührt. Dann wurde nach heißer Filtration das Ligroin abdestilliert.
Man erhielt 14,2 g (80 % der Theorie) 2-[4-(4-Trifluor-methyl)-phenoxy)-phenoxy]-thiopropionsäuremethylester.
Das Produkt wurde über eine Kieselgelsäule mit einem Laufmittel aus Hexan und Aceton = 9:1 chromatographisch gereinigt.
$n_D^{21} = 1,5382$.
Nach der im Beispiel 1 angegebenen Methode wurden auch die in der folgenden Tabelle 8 formelmäßig aufgeführten Stoffe hergestellt.

**Tabelle 8**

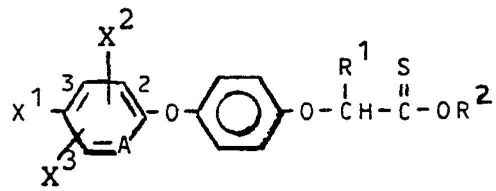

| Beisp.-Nr. | A | $X^1$ | $X^2$ | $X^3$ | $R^1$ | $R^2$ | Schmelzp. bzw. Brechungsindex |
|---|---|---|---|---|---|---|---|
| 2 | -CH | Cl | 2-$CF_3$ | H | $CH_3$ | $CH_3$ | $n_D^{21}$:1,5499 |
| 3 | -CH | H | 3-$CF_3$ | H | $CH_3$ | $C_2H_5$ | $n_D^{21}$:1,5307 |
| 4 | -N | $CF_3$ | H | H | $CH_3$ | $CH_3$ | $n_D^{21,5}$:1,5396 |
| 5 | -N | Cl | 2-Cl | H | $CH_3$ | $CH_3$ | $n_D^{21,5}$:1,5966 |
| 6 | -CH | $CF_3$ | H | H | $CH_3$ | $C_2H_5$ | $n_D^{20,5}$:1,5307 |
| 7 | -N | $CF_3$ | H | H | $CH_3$ | $C_2H_5$ | F : 80°C |
| 8 | -N | Cl | 2-Cl | H | $CH_3$ | $C_2H_5$ | $n_D^{20,5}$:1,5824 |
| 9 | -CH | Cl | 2-Cl | H | $CH_3$ | $CH_3$ | $n_D^{21,5}$:1,5925 |
| 10 | -N | $CF_3$ | 2-Cl | H | $CH_3$ | $CH_2-CH_2-OC_2H_5$ | $n_D^{20}$ =1,5229 |

In dem nachfolgend beschriebenen biologischen Test wurde die folgende Verbindung als Vergleichssubstanz eingesetzt:

(A) = 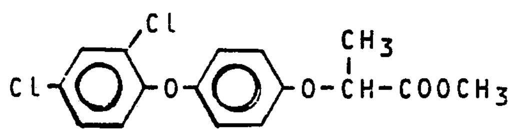

2-[4-(2,4-Dichlorphenoxy)-phenoxy]-propionsäuremethyl-ester (bekannt aus DE-OS 2 223 894).

**Beispiel A**

Post-emergence-Test
Lösungsmittel: 5 Gewichtsteile Aceton
Emulgator: 1 Gewichtsteil Alkylarylpolyglykolether
Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 1 Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung.

In diesem Test zeigen die erfindungsgemäßen Wirkstoffe (1), (4) und (5) bei der Bekämpfung von Alopecurus, Echinochloa und Setaria in Zuckerrüben bei einer Aufwandmenge von 0,5 kg Wirkstoff pro Hektar eine bessere selektive herbizide Wirksamkeit als die Vergleichssubstanz (A).

**Patentansprüche**

1. Substituierte Alkanthiocarbonsäure-Derivate der Formel

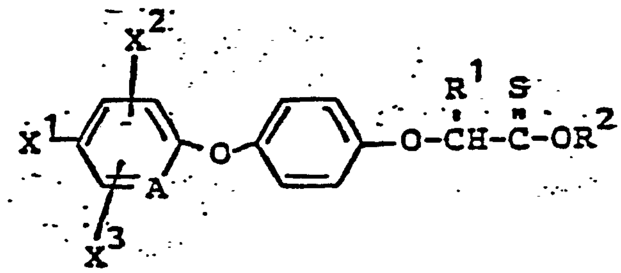

in welcher
A für Stickstoff oder eine -CH-Gruppierung steht,
$X^1$ für Wasserstoff, Trifluormethyl oder Chlor steht,
$X^2$ für Wasserstoff, Trifluormethyl oder Chlor steht,
$X^3$ für Wasserstoff, Trifluormethyl oder Chlor steht,
$R^1$ für Wasserstoff oder Methyl steht und
$R^2$ für Wasserstoff, gegebenenfalls durch Hydroxy, Halogen, $C_1$-$C_4$-Alkoxy, $C_1$-$C_4$-Alkylthio, $C_3$-$C_6$-Cycloalkyl, Amino, Alkylamino mit 1 bis 4 Kohlenstoffatomen, Dialkylamino mit 1 bis 4 Kohlenstoffatomen pro Alkylrest und/oder über Stickstoff gebundene, gesättigte, 5- oder 6-gliedrige Reterocyclen, die bis zu 3 Stickstoff- und/oder Sauerstoffatome enthalten, substituiertes Alkyl mit 1 bis 6 Kohlenstoffatomen,
für gegebenenfalls durch Fluor, Chlor, Brom und/oder lod substituiertes Alkenyl,
für gegebenenfalls durch Fluor, Chlor, Brom und/oder lod substituiertes Alkinyl,
für gegebenenfalls durch Halogen, $C_1$-$C_4$-Alkyl, $C_1$-$C_4$-Alkylthio, $C_1$-$C_4$-Alkoxy, Cyano und/oder Nitro substituiertes Phenyl,
für gegebenenfalls durch Halogen, $C_1$-$C_4$-Alkyl, $C_1$-$C_4$-Alkylthio, $C_1$-$C_4$-Alkoxy, Cyano und/oder Nitro substituiertes Benzyl,
für gegebenenfalls im Phenylteil durch Halogen, $C_1$-$C_4$-Alkyl, $C_1$-$C_4$-Alkylthio, $C_1$-$C_4$-Alkoxy, Cyano und/oder Nitro substituiertes phenylethyl,
für gegebenenfalls im phenylteil durch Halogen, $C_1$-$C_4$-Alkyl, $C_1$-$C_4$-Alkylthio, $C_1$-$C_4$-Alkoxy, Cyano und/oder Nitro substituiertes phenyl-($C_1$-$C_4$)-alkoxy-($C_1$-$C_4$)-Alkyl oder
für gegebenenfalls durch Alkyl mit 1 bis 4 Kohlenstoffatomen und/oder Halogen substituiertes Cycloalkylmit 3-bis-6 Kohlenstoffatomen steht.

2. Verfahren zur Herstellung von substituierten Alkanthiocarbonsäure-Derivaten der Formel

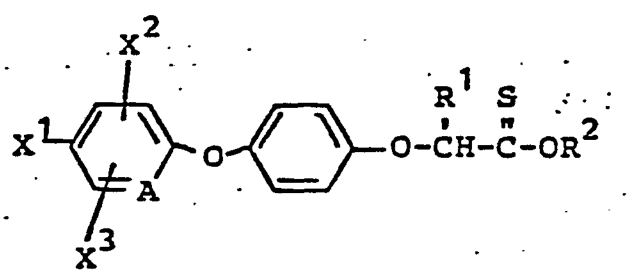

(I)

in welcher
A für Stickstoff oder eine CH-Gruppierung steht,
$X^1$ für Wasserstoff, Trifluormethyl oder Chlor steht,
$X^2$ und $X^3$ unabhängig voneinander für Wasserstoff, Trifluormethyl oder Chlor stehen,
$R^1$ für Wasserstoff oder Methyl steht und
$R^2$ für Wasserstoff, gegebenenfalls durch Hydroxy, Halogen, $C_1$-$C_4$-Alkoxy, $C_1$-$C_4$-Alkylthio, $C_3$-$C_6$-Cycloalkyl, Amino, Alkylamino mit 1 bis 4 Kohlenstoffatomen, Dialkylamino mit 1 bis 4 Kohlenstoffatomen pro Alkylrest und/oder über Stickstoff gebundene, gesättigte, 5- oder 6-gliedrige Heterocyclen, die bis zu 3 Stickstoff- und/oder Sauerstoffatome enthalten, substituiertes Alkyl mit 1 bis 6 Kohlenstoffatomen,
für gegebenenfalls durch Fluor, Chlor, Brom und/ oder lod substituiertes Alkenyl,

für gegebenenfalls durch Fluor, Chlor, Brom und/ oder Iod substituiertes Alkinyl,

für gegebenenfalls durch Halogen, $C_1$-$C_4$-Alkyl, $C_1$-$C_4$-Alkylthio, $C_1$-$C_4$-Alkoxy, Cyano und/oder Nitro substituiertes Phenyl,

für gegebenenfalls durch Halogen, $C_1$-$C_4$-Alkyl, $C_1$-$C_4$-Alkylthio, $C_1$-$C_4$-Alkoxy, Cyano und/oder Nitro substituiertes Benzyl,

für gegebenenfalls im phenylteil durch Halogen, $C_1$-$C_4$-Alkyl, $C_1$-$C_4$-Alkylthio, $C_1$-$C_4$-Alkoxy, Cyano und/oder Nitro substituiertes phenylethyl,

für gegebenenfalls im Phenylteil durch Halogen, $C_1$-$C_4$-Alkyl, $C_1$-$C_4$-Alkylthio, $C_1$-$C_4$-Alkoxy, Cyano und/oder Nitro substituiertes Phenyl-($C_1$-$C_4$)-alkoxy-($C_1$-$C_4$-)-Alkyl oder

für gegebenenfalls durch Alkyl mit 1 bis 4 Kohlenstoffatomen und/oder Halogen substituiertes Cycloalkyl mit 3 bis 6 Kohlenstoffatomen steht.

dadurch gekennzeichnet, daß man Phenoxyalkancarbonsäure-Derivate der Formel

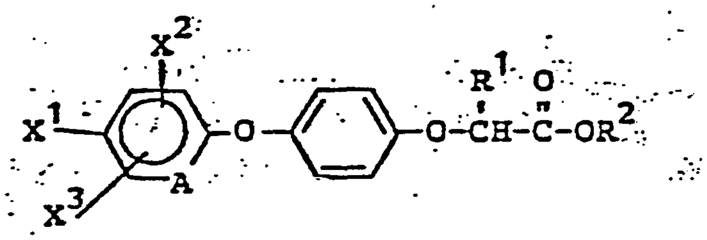

(II)

in welcher

A, $X^1$, $X^2$, $X^3$, $R^1$ und $R^2$ die oben angegebene Bedeutung haben,

mit Schwefelungsmitteln gegebenenfalls in Gegenwart eines Verdünnungsmittels umsetzt.

3. Herbizide Mittel, gekennzeichnet durch einen Gehalt an mindestens einem substituierten Alkanthiocarbonsäure-derivat der Formel (I) gemäß Anspruch 1 und 2.

4. Verwendung von substituierten Alkanthiocarbonsäure-Derivaten der Formel (I) gemäß Anspruch 1 und 2 zur Bekämpfung von Unkräutern.

5. Verfahren zur Bekämpfung von Unkräutern, dadurch gekennzeichnet, daß man substituierte Alkanthiocarbonsäure-Derivate der Formel (I) gemäß Anspruch 1 und 2 auf die Unkräuter und/oder deren Lebensraum ausbringt.

6. Verfahren zur Herstellung von herbiziden Mitteln, dadurch gekennzeichnet, daß man substituierte Alkanthiocarbonsäure-Derivate der Formel (I) gemäß Anspruch 1 und 2 mit Streckmitteln und/oder oberflächenaktiven Stoffen vermischt.

7. Substituiertes Alkanthiocarbonsäure-Derivat der Formel

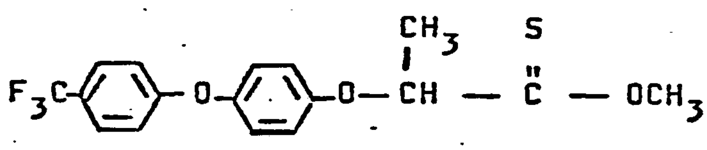

8. Substituiertes Alkanthiocarbonsäure-Derivat der Formel

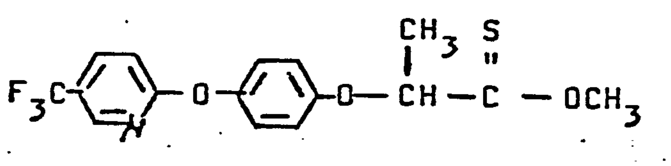

9. Substituiertes Alkanthiocarbonsäure-Derivat der Formel

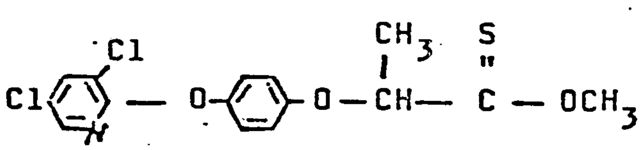

**Claims**

1. Substituted alkanethiocarboxylic acid derivatives of the formula

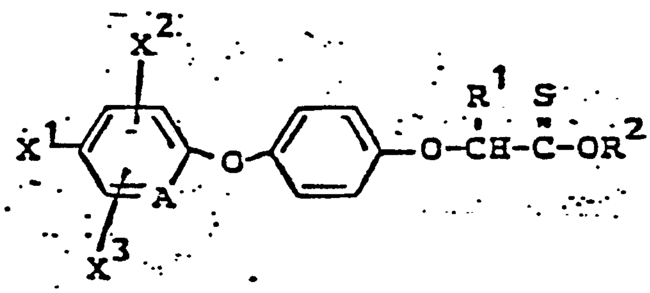

in which
A represents nitrogen or a -CH grouping,
$X^1$ represents hydrogen, trifluoromethyl or chlorine,
$X^2$ represents hydrogen, trifluoromethyl or chlorine,
$X^3$ represents hydrogen, trifluoromethyl or chlorine,
$R^1$ represents hydrogen or methyl and
$R^2$ represents hydrogen, alkyl which has 1 to 6 carbon atoms and is optionally substituted by hydroxyl, halogen, $C_1$-$C_4$-alkoxy, $C_1$-$C_4$-alkylthio, $C_3$-$C_6$-cycloalkyl, amino, alkylamino with 1 to 4 carbon atoms, dialkylamino with 1 to 4 carbon atoms per alkyl radical and/or via nitrogen,
alkenyl which is optionally substituted by fluorine, chlorine, bromine and/or iodine,
alkinyl which is optionally substituted by fluorine, chlorine, bromine and/or iodine,
phenyl which is optionally substituted by halogen, $C_1$-$C_4$-alkyl, $C_1$-$C_4$-alkylthio, $C_1$-$C_4$-alkoxy, cyano and/or nitro,
benzyl which is optionally substituted by halogen, $C_1$-$C_4$-alkyl, $C_1$-$C_4$-alkylthio, $C_1$-$C_4$-alkoxy, cyano and/or nitro,
phenylethyl which is optionally substituted in the phenyl part by halogen, $C_1$-$C_4$-alkyl, $C_1$-$C_4$-alkylthio, $C_1$-$C_4$-alkoxy, cyano and/or-nitro,
phenyl-($C_1$-$C_4$)-alkoxy-($C_1$-$C_4$)-alkyl which is optionally substituted in the phenyl part by halogen, $C_1$-$C_4$-alkyl, $C_1$-$C_4$-alkylthio, $C_1$-$C_4$-alkoxy, cyano and/or nitro, or
cycloalkyl which has 3 to 8 carbon atoms and is optionally substituted by alkyl with 1 to 4 carbon atoms and/or halogen.

2. Process for the preparation of substituted alkanethiocarboxylic acid derivatives of the formula

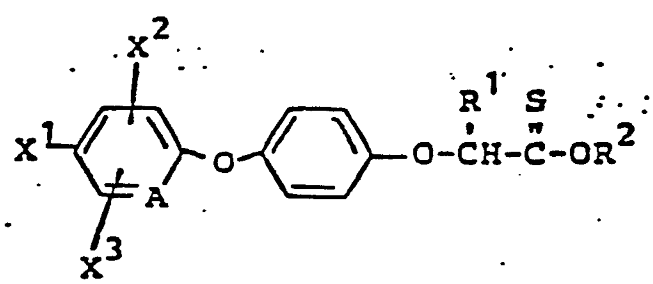

in which
A represents nitrogen or a CH grouping,
$X^1$ represents hydrogen, trifluoromethyl or chlorine,
$X^2$ and $X^3$ independently of one another represent hydrogen, trifluoromethyl or chlorine,
$R^1$ represents hydrogen or methyl and
$R^2$ represents hydrogen, alkyl which has 1 to 6 carbon atoms and is optionally substituted by hydroxyl, halogen, $C_1$-$C_4$-alkoxy, $C_1$-$C_4$-alkylthio, $C_3$-$C_6$-cycloalkyl, amino, alkylamino with 1 to 4 carbon atoms, dialkylamino with 1 to 4 carbon atoms per alkyl radical and/or saturated 5- or 6-membered heterocycles which contain up to 3 nitrogen and/or oxygen atoms and are bonded via nitrogen,
alkenyl which is optionally substituted by fluorine, chlorine, bromine and/or iodine,
alkinyl which is optionally substituted by fluorine, chlorine, bromine and/or iodine,
phenyl which is optionally substituted by halogen, $C_1$-$C_4$-alkyl, $C_1$-$C_4$-alkylthio, $C_1$-$C_4$-alkoxy, cyano and/or nitro,

benzyl which is optionally substituted by halogen, $C_1$-$C_4$-alkyl, $C_1$-$C_4$-alkylthio, $C_1$-$C_4$-alkoxy, cyano and/or nitro,

phenylethyl which is optionally substituted in the phenyl part by halogen, $C_1$-$C_4$-alkyl, $C_1$-$C_4$-alkylthio, $C_1$-$C_4$-alkoxy, cyano-and/or nitro,

phenyl-($C_1$-$C_4$)-alkoxy-($C_1$-$C_4$)-alkyl which is optionally substituted in the phenyl part by halogen, $C_1$-$C_4$-alkyl, $C_1$-$C_4$-alkylthio, $C_1$-$C_4$-alkoxy, cyano and/or nitro, or

cycloalkyl which has 3 to 6 carbon atoms and is optionally substituted by alkyl with 1 to 4 carbon atoms and/or halogen,

characterised in that phenoxyalkanecarboxylic acid derivatives of the formula

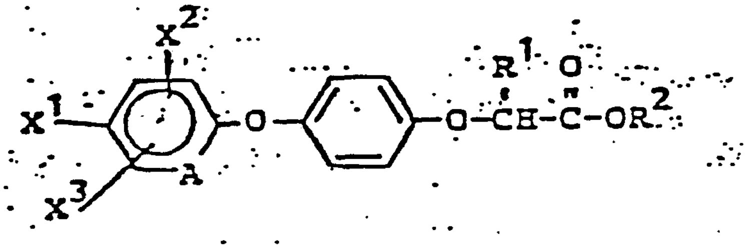
(II)

in which

A $X^1$, $X^2$, $X^3$, $R^1$ and $R^2$ have the above mentioned meaning,

are reacted with sulphurising agents, if appropriate in the presence of a diluent.

3. Herbicidal agents, characterised in that they contain at least one substituted alkane thiocarboxylic acid derivative of the formula (I) according to Claim 1 and 2.

4. Use of substituted alkanethiocarboxylic acid derivatives of the formula (I) according to Claim 1 and 2 for combating weeds.

5. Method of combating weeds, characterised in that substituted alkanethiocarboxylic acid derivatives of the formula (I) according to Claim 1 and 2 are applied to the weeds and/or their environment.

6. Process for the preparation of herbicidal agents, characterised in that substituted alkanethiocarboxylic acid derivatives of the formula (I) according to Claim 1 and 2 are mixed with extenders and/or surface-active substances.

7. Substituted alkanethiocarboxylic acid derivative of the formula

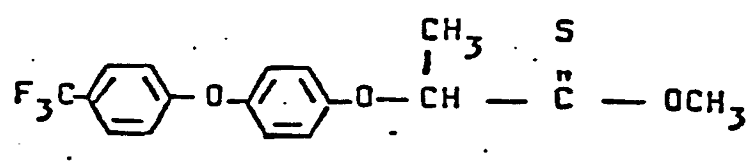

8. Substituted alkanethiocarboxylic acid derivative of the formula

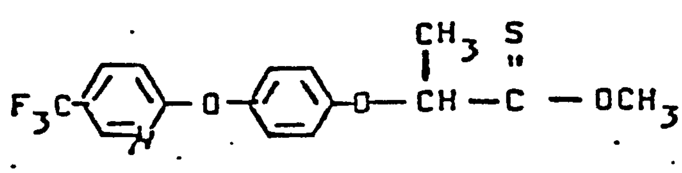

9. Substituted alkanethiocarboxylic acid derivative of the formula

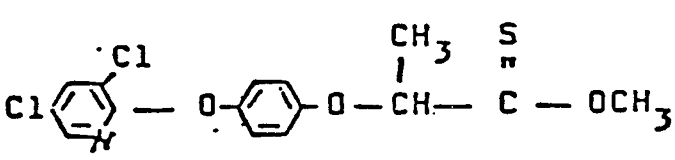

**Revendications**

1. Dérivés d'acides alcanethiocarboxyliques substitués de formule

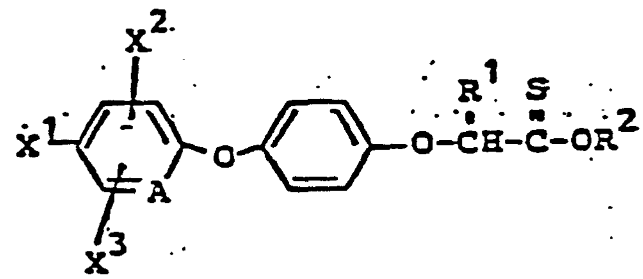

dans laquelle
A est de l'azote ou un groupement -CH-,
$X^1$ est de l'hydrogène un groupe trifluorométhyle ou du chlore,
$X^2$ est de l'hydrogène, un groupe trifluorométhyle ou du chlore,
$X^3$ est de l'hydrogène, un groupe trifluorométhyle ou du chlore,
$R^1$ est de l'hydrogène ou un groupe méthyle, et
$R^2$ est de l'hydrogène, un groupe alkyle de 1 à 6 atomes de carbone éventuellement substitué par un radical hydroxy, halogéno, alkoxy en $C_1$ à $C_4$, alkylthio en $C_1$ à $C_4$, cycloalkyle en $C_3$ à $C_6$, amino, alkylamino ayant 1 à 4 atomes de carbone, dialkylamino ayant 1 à 4 atomes de carbone par reste alkyle et/ou des hétérocycles pentagonaux ou hexagonaux saturés attachés par de l'azote, qui contiennent jusqu'à 3 atomes d'azote et/ou d'oxygène,
un groupe alcényle éventuellement substitué par du fluor, du chlore, du brome et/ou de l'iode,
un groupe alcynyle éventuellement substitué par du fluor, du chlore, du brome et/ou de l'iode,
un groupe phényle éventuellement substitué par un radical halogéno, alkyle en $C_1$ à $C_4$, alkylthio en $C_1$ à $C_4$, alkoxy en $C_1$ à $C_4$, cyano et/ou nitro,
un groupe benzyle éventuellement substitué par un radical halogéno, alkyle en $C_1$ à $C_4$, alkylthio en $C_1$ à $C_4$, alkoxy en $C_1$ à $C_4$, cyano et/ou nitro,
un groupe phényléthyle éventuellement substitué dans la partie phényle par un radical halogéno, alkyle en $C_1$ à $C_4$, alkylthio en $C_1$ à $C_4$, alkoxy en $C_1$ à $C_4$, cyano et/ou nitro, un groupe phényl-(alkoxy en $C_1$ à $C_4$)-(alkyle en $C_1$ à $C_4$) éventuellement substitué dans la partie phényle par un radical halogéno,
alkyle en $C_1$ à $C_4$, alkylthio en $C_1$ à $C_4$, alkoxy en $C_1$ à $C_4$, cyano et/ou nitro ou
un groupe cycloalkyle de 3 à 6 atomes de carbone éventuellement substitué par un radical- alkyle de 1 à 4 atomes de carbone et/ou par un radical halogéno.

2. Procédé de production de dérivés d'acides alcanethiocarboxyliques substitués de formule

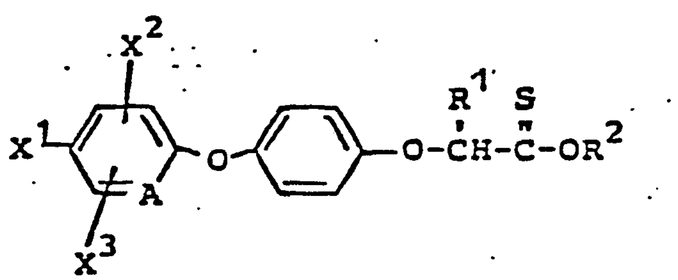

(I)

dans laquelle
A est de l'azote ou un groupement -CH-,
$X^1$ est de l'hydrogène, un groupe trifluorométhyle ou du chlore,
$X^2$ et $X^3$ représentent, indépendamment l'un de l'autre, de l'hydrogène, un groupe trifluorométhyle ou du chlore,
$R^1$ est de l'hydrogène ou un groupe méthyle, et
$R^2$ est de l'hydrogène, un groupe alkyle de 1 à 6 atomes de carbone éventuellement substitué par un radical hydroxy, halogéno, alkoxy en $C_1$ à $C_4$, alkylthio en $C_1$ à $C_4$, cycloalkyle en $C_3$ à $C_6$, amino, alkylamino ayant 1 à 4 atomes de carbone, dialkylamino ayant 1 à 4 atomes de carbone par reste alkyle et/ou des hétérocycles pentagonaux ou hexagonaux saturés attachés par de l'azote, qui contiennent jusqu'à 3 atomes d'azote et/ou d'oxygène,
un groupe alcényle éventuellement substitué par du fluor, du chlore, du brome et/ou de l'iode,
un groupe alcynyle éventuellement substitué par du fluor, du chlore, du brome et/ou de l'iode,

un groupe phényle éventuellement substitué par un radical halogèno, alkyle en $C_1$ à $C_4$, alkylthio en $C_1$ à $C_4$, alkoxy en $C_1$ à $C_4$, cyano et/ou nitro,

un groupe benzyle éventuellement substitué par un radical halogéno, alkyle en $C_1$ à $C_4$, alkylthio en $C_1$ à $C_4$, alkoxy en $C_1$ à $C_4$, cyano et/ou nitro,

un groupe phényléthyle éventuellement substitué dans la partie phényle par un radical halogéno, alkyle en $C_1$ à $C_4$, alkylthio en $C_1$ à $C_4$, alkoxy en $C_1$ à $C_4$, cyano et/ou nitro,

un groupe phényl-(alkoxy en $C_1$ à $C_4$)-(alkyle en $C_1$ à $C_4$) éventuellement substitué dans la partie phényle par un radical halogéno, alkyle en $C_1$ à $C_4$, alkylthio en $C_1$ à $C_4$, alkoxy en $C_1$ à $C_4$, cyano et/ou nitro ou

un groupe cycloalkyle de 3 à 6 atomes de carbone éventuellement substitué par un radical alkyle de 1 à 4 atomes de carbone et/ou par un radical halogéno,

caractérisé en ce qu'on fait réagir des dérivés d'acides phénoxyalcanecarboxyliques de formule

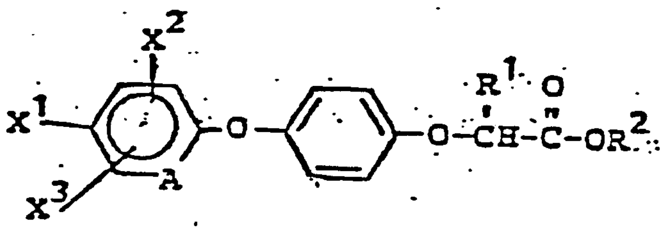

(II)

dans laquelle

A $X^1$ $X^2$ $X^3$ $R^1$ et $R^2$ ont la définition indiquée ci-dessus,

avec des agents de sulfuration, éventuellement en présence d'un diluant.

3. Compositions herbicides, caractérisées par une teneur en au moins un dérivé d'acide alcanethiocarboxylique substitué de formule (I) suivant les revendications 1 et 2.

4. Utilisation de dérivés d'acides alcanethiocarboxyliques substitués de formule (I) suivant les revendications 1 et 2 pour combattre des mauvaises herbes.

5. Procédé pour combattre des mauvaises herbes, caractérisé en ce qu'on fait agir des dérivés d'acides alcanethiocarboxyliques substitués de formule (I) suivant les revendications 1 et 2 sur les mauvaises herbes et/ou sur leur milieu.

6. Procédé de préparation de compositions herbicides, caractérisé en ce qu'on mélange des dérivés d'acides alcanethiocarboxyliques substitués de formule (I) suivant les revendications 1 et 2 avec des diluants et/ou des agents tensio-actifs.

7. Dérivé d'acide alcanethiocarboxylique substitué de formule

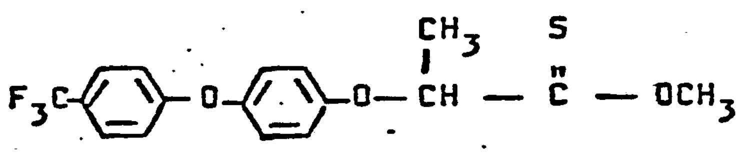

8. Dérivé d'acide alcanethiocarboxylique substitué de formule

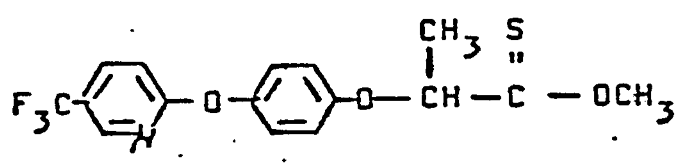

9. Dérivé d'acide alcanethiocarboxylique substitué de formule